Europäisches Patentamt

European Patent Office

Office européen des brevets

(19)

(11) Veröffentlichungsnummer: **0 296 635**
**A2**

(12) # EUROPÄISCHE PATENTANMELDUNG

(21) Anmeldenummer: 88110208.1

(22) Anmeldetag: 27.06.88

(51) Int. Cl.⁴: **A61B 17/12**

(30) Priorität: 26.06.87 AT 1622/87

(43) Veröffentlichungstag der Anmeldung:
**28.12.88 Patentblatt 88/52**

(84) Benannte Vertragsstaaten:
**AT DE FR NL SE**

(71) Anmelder: **SIEMENS AKTIENGESELLSCHAFT ÖSTERREICH**
**Siemensstrasse 88-92**
**A-1210 Wien(AT)**

(84) **AT**

(71) Anmelder: **Siemens Aktiengesellschaft Berlin und München**
**Wittelsbacherplatz 2**
**D-8000 München 2(DE)**

(84) **DE FR NL SE**

(72) Erfinder: **Egkher, Emmerich Dr.**
**Vortgartenstrasse 44/31**
**A-1200 Wien(AT)**
Erfinder: **Wielke, Bernhard Dr.**
**Jessengasse 2**
**A-1190 Wien(AT)**
Erfinder: **Freund, Rudolf, Dipl.Ing.Dr.**
**Kolpingstrasse 56**
**A-2000 Stockerau(AT)**

(54) **Verfahren zum Betrieb einer Blutsperre.**

(57) Der notwendige Überdruck zur Blutsperre einer Körperextremität während der Operation wird möglichst gering gehalten, wenn der Druck der Blutsperrmanschette dem aktuellen systolischen Blutdruck nachgeführt wird. Der Überdruck hängt vom Gewebsturgor, Geschlecht, Extremitätenumfang und Alter der operierten Person und der Dauer der Blutsperre ab. Die Regelung erfolgt computergesteuert, und über die Ein-/Ausgabevorrichtung (EA) werden die Zahlenwerte der Parameter menügeführt eingegeben. Die Blutsperre wird durch Betätigen der Blutsperrtaste (BST) aktiviert und durch gleichzeitige Betätigung zweier Lösetasten (LT) beendet. Ein versehentliches Aufheben der Blutsperre ist somit nahezu ausgeschlossen. Wichtige Zahlenwerte werden mit Leuchtdioden an der Eingabeanzeige (LED-D) und Überwachungsanzeige (LED-Ü) angezeigt. Die Bedienerführung und Überwachungsangaben erfolgen im Klartext über die Flüssigkristallanzeige (LCD-B), um die Aufmerksamkeit des Bedienungspersonals nicht von der Anzeige mit Leuchtdioden abzulenken. Die Vorrichtung verfügt über eine eigene Druckversorgung und ist auch für den mobilen Einsatz im Krankenwagen oder am Unfallsort ausgerüstet. Die Wahrscheinlichkeit von Gewebeschädigungen durch Abbinden ist dadurch weitgehend herabgesetzt.

FIG 2

## Verfahren zum Betrieb einer Blutsperre

Die Erfindung betrifft ein Verfahren zum Betrieb einer Blutsperre für Körperextremitäten, wobei der Blutdruck gemessen, dieser Meßwert digitalisiert und einem Mikrocomputer zugeleitet wird, von dem eine Druckregelung der Blutsperre auf einen von den Parametern Gewebsturgor, Geschlecht, Extremitätenumfang und Alter der Person und Dauer der Blutsperre abhängigen Druckwert über dem aktuellen systolischen Blutdruck eingestellt wird.

Dieses Verfahren wird in der Zeitschrift Unfallchirurgie 12 (1986), Seite 200 - 203, (Nr. 4) beschrieben. Der Überdruck wird für jeden Patienten individuell festgelegt und ist so klein, daß er bei diesem Patienten eine gerade noch wirksame Blutsperre bewirkt. Daher kann die Sperrzeit deutlich verlängert werden, ohne daß eine Schädigung des unter einer Blutsperrmanschette liegenden Gewebes zu erwarten ist. Der Druck der Blutsperre wird dem sich während der Operation ändernden Blutdruck des Patienten nachgeführt und mit Fortdauer der Operation in Abhängigkeit vom Gewebsturgor der Extremität des Patienten weiter abgesenkt. Da das Verfahren für Forschungszwecke konzipiert ist, wird auf viele Eingriffsmöglichkeiten, selbst während der Operation, Wert gelegt. Zu diesem Zweck sind eine vollständige alphanumerische Tastatur und ein Bildschirm vorhanden.

Die US 4, 479, 494 zeigt eine Vorrichtung zur Blutsperre einer Körperextremität mit einer Blutsperrmanschette. Sie wird durch die Betätigung mehrerer Kippschalter bedient, und die Anzeige von eingestellten Werten erfolgt über zwei dreistellige Leuchtdiodenfelder. Verschiedene Funktionen werden durch Kontrolleuchten angezeigt. Diese Blutsperre ermöglicht es, einen bestimmten Überdruck an der Blutsperrmanschette einzustellen, der dann während der Operation konstant über dem aktuellen systolischen Blutdruck gehalten wird. Die Wahl dieses Wertes ist aber verhältnismäßig willkürlich. Eine Eingabe und Berücksichtigung von patientenspezifischen Daten ist nicht möglich.

Der Erfindung liegt die Aufgabe zugrunde, das Verfahren an klinische Anforderungen anzupassen und die Betriebs- und Bedienungssicherheit zu erhöhen.

Dies wird gemäß Patentanspruch 1 dadurch erreicht, daß die Zahlenwerte der Parameter des Druckwertes dem Mikrocomputer menügeführt eingegeben werden, die Blutsperre durch Betätigung einer einzigen Blutsperrtaste aktiviert und durch gleichzeitige Betätigung zweier Lösetasten beendet wird und daß Überwachungssignale und Eingabewerte mit Leuchtdioden, Bedienungshinweise alphanumerisch mit Flüssigkristallen angezeigt werden.

Dadurch wird die Menge der zur Bedienung der Blutsperre einzugebenden Daten auf die Zahlenwerte der Parameter begrenzt. Die Abfrage dieser Werte schließt eine Fehlbedienung nahezu aus. Um ein versehentliches Öffnen der Blutsperre zu erschweren, kann diese Funktion als einzige nur durch das gleichzeitige Betätigen zweier Tasten ausgeführt werden. Eine räumliche Trennung von Funktions- und Eingabetasten erhöht die Übersichtlichkeit und Bedienungssicherheit. Eine Anzeige der für den Gesundheitszustand des Patienten wichtigen Zahlenwerte durch Leuchtdioden ist besonders augenfällig, um dadurch des Bedienungspersonal gegebenenfalls zu warnen. Die Flüssigkristallanzeige wird gewählt, um im Klartext lesbare Bedienungshinweise zu geben und die Aufmerksamkeit des Bedienungspersonals nicht von der Anzeige mit Leuchtdioden abzulenken.

Während der Operation wird vor einer möglichen Schädigung des Gewebes unter der Blutsperrmanschette und der abgebundenen Extremität dadurch gewarnt, daß die maximale Dauer der Blutsperre ohne Schädigung des betroffenen Körpergewebes in Abhängigkeit von der Größe des Druckwertes vom Mikrocomputer berechnet und durch ein entsprechendes Überwachungssignal angezeigt wird.

Von einem Überwachungssignal wird die Aktivierung der Blutsperre angezeigt, um auch dem Operateur ohne Hilfe des Bedienungspersonals der Blutsperre deren Wirkung anzuzeigen. Die versehentliche Betätigung einer Taste wird dadurch vermieden, daß ein Tastendruck erst nach einer vorgegebenen Mindestdauer registriert wird. Somit wird das bewußte Drücken der Tasten im Sekundenbereich notwendig, um eine Funktion auszulösen.

Zur Durchführung des Verfahrens ist ein Blutdruckmesser über einen Analog/Digital-Wandler mit einem Dateneingang des Mikrocomputers verbunden, der Mikrocomputer über einen Steuerausgang mit dem Ansteuerteil einer elektrisch betriebenen Pumpe und über eine Datenschnittstelle mit einer Ein-/Ausgabevorrichtung verbunden, die aus einem Tastenfeld, einer Eingabeanzeige aus Leuchtdioden, einer Überwachungsanzeige aus Leuchtdioden und einer Flüssigkristallanzeige für Bedienerführung und Überwachungsangaben besteht, und ist der Druckteil der Pumpe mit einer Blutsperrmanschette verbunden.

Mit dem Mikrocomputer kann überprüft werden, ob die Eingabewerte innerhalb plausibler Grenzen liegen. Die Pumpe zur Erzeugung des Drucks in der Blutsperrmanschette ist in das Steuer- und Regelungsgerät integriert. Dadurch ist

die Blutsperre von einer in manchen Spitälern vorhandenen externen Druckversorgung unabhängig. Darüberhinaus ist die Stärke der Pumpe so zu bemessen, daß ein kritischer Druck in der Blutsperrmanschette nicht überschritten werden kann. Anderenfalls ist ein Überdruckventil vorzusehen. Dadurch wird die Zerstörung des unter der Manschette liegenden Gewebes durch zu starke Pressung vermieden. Zum Druckaufbau wird im allgemeinen Luft verwendet. Diese Vorrichtung zur Blustperre kann transporta bel ausgeführt werden, wodurch auch ein Einsatz im Krankenwagen oder am Unfallort möglich ist. Dadurch wird der Blutverlust von Opfern bei Verkehrs- und Sportunfällen mit Verletzungen an Armen oder Beinen bis zur Einlieferung ins Krankenhaus verringert. Die beim bisher üblichen Abbinden auftretenden Gewebeschädigungen an den Extremitäten können so wesentlich verringert oder sogar vermieden werden.

Um den automatisch eingestellten und gehaltenen Druck in der Blutsperrmanschette einfach korrigieren zu können, sind zwei Schrittasten zur Änderung des eingestellten Überdruckes in vorgegebenen Schritten an der Ein-/Ausgabevorrichtung vorhanden. Zwischen Pumpe und Blutsperrmanschette ist ein Rückschlagventil eingebaut, um bei plötzlichem Defekt der Pumpe ein unbeabsichtigtes Absinken des Drucks der Blutsperrmanschette zu verhindern.

Bei Stromausfall oder Pumpendefekt wird die Funktionstüchtigkeit der Blutsperre dadurch erhalten, daß zusätzlich zur Pumpe eine Handpumpe mit Kontrollmanometer mit der Blutsperrmanschette verbunden ist. Die Wirkung der Blutsperre bleibt beim Auftreten eines Lecks erhalten, wenn die Blutsperrmanschette in zwei voneinander unabhängige ringförmige Kammern unterteilt ist. Zur Dokumentation beispielsweise des Blutdrucks und des Drucks in der Blutsperrmanschette ist ein Aufzeichnungsgerät mit einem Datenausgang des Mikrocomputers verbunden.

Die Erfindung wird anhand eines Ausführungsbeispieles und von Zeichnungen näher erläutert. Es zeigen:

Fig. 1 ein schematisches Blockschaltbild des Ausführungsbeispieles und

Fig. 2 eine Darstellung des Bedienungs- und Anzeigefeldes des Steuer- und Regelteils.

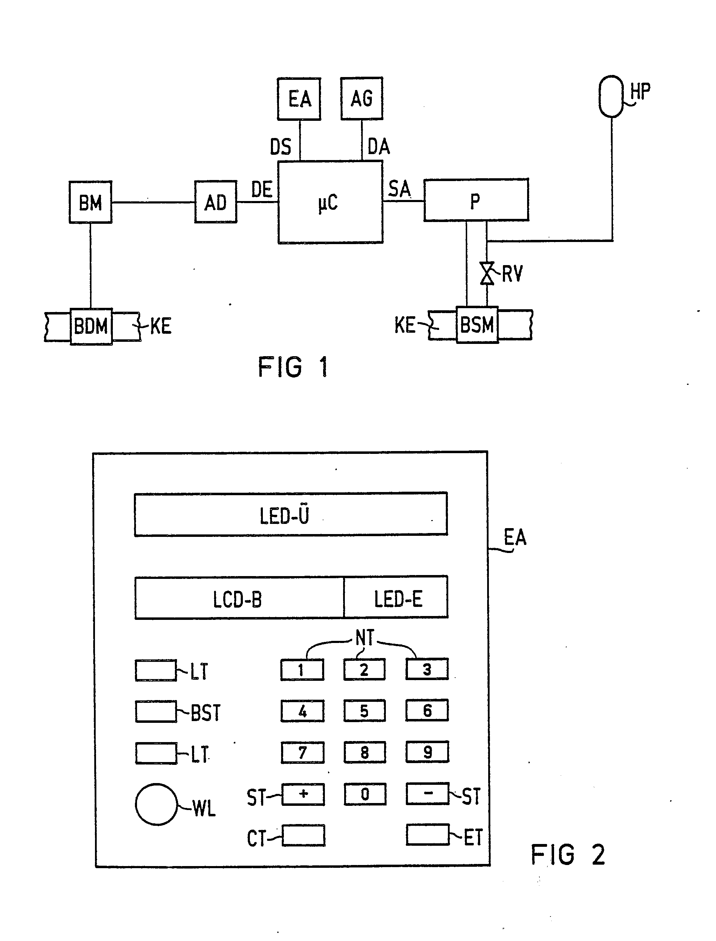

Eine Körperextremität KE eines Patienten wird während der Operation zur Verringerung des Blutverlustes und zum Freihalten des Operationsgebietes von Blut mit einer Vorrichtung zur·Blutsperre abgebunden. Wie Fig. 1 zeigt, ist ein nichtinvasiver Blutdruckmesser BM über einen Analog/Digital-Wandler AD mit der Vorrichtung verbunden. Die Blutdruckmanschette BDM des Blutdruckmessers BM ist an einer anderen Körperextremität KE des Patienten angelegt. Die Vorrichtung zur Blutsperre enthält einen Mikrocomputer μC zur Steuerung des Druckes einer Blutsperrmanschette BSM an der zu operierenden Körperextremität KE. Dazu ist der Mikrocomputer μC über einen Steuerausgang SA mit dem Ansteuerteil einer elektrisch betriebenen Pumpe P verbunden. Diese pumpt über eine mit einem Rückschlagventil RV gesicherte Leitung die Blutsperrmanschette BSM mit Luft auf. Über eine Rückleitung kann mit einem Ventil steuerbar Luft aus der Blutsperrmanschette BSM abgelassen werden. Eine Handpumpe HP mit mechanischem Kontrollmanometer sorgt für das Aufrechterhalten der Blutsperre bei einem Ausfall einer Komponente der Vorrichtung.

Dem Mikrocomputer μC wird über einen Dateneingang DE der aktuelle Blutdruckwert des Patienten zugeführt. In Abhängigkeit von den personenbezogenen Daten des Gewebsturgors, Geschlechtes, Extremitätenumfanges und Alters und der seit dem Einsetzen der Blutsperre vergangenen Zeit errechnet der Mikrocomputer μC den momentan geringstmöglichen Überdruck zum Aufrechterhalten der Blutsperre. Dieser Druck, etwa 20 - 50 mm Hg, wird zum Wert des systolischen Blutdruckes addiert und die Blutsperrmanschette BSM über die Pumpe P auf diesem Gesamtdruck gehalten. Zur Dokumentation dieser Werte während der Operation ist der Mikrocomputer μC über einen Datenausgang DA mit einem Aufzeichnungsgerät AG verbunden. Dieses Aufzeichnungsgerät AG kann beispielsweise ein Drucker oder Plotter aber auch ein Datenspeicher oder Computer sein. Die Eingabe der personenbezogenen Daten und die Ausgabe von Überwachungswerten wie Blutdruck, Datum oder Uhrzeit erfolgt über eine Ein-/Ausgabe vorrichtung EA. Diese ist über eine Datenschnittstelle DS mit dem Mikrocomputer μC verbunden.

Fig. 2 zeigt das Bedienungs- und Anzeigefeld der Ein-/Ausgabevorrichtung EA. Vor dem Einsatz zur Blutsperre sind die personenbezogenen Daten des Patienten einzugeben. Dazu wird durch Drücken einer Eingabetaste ET die Möglichkeit der Dateneingabe aktiviert. Als Bedienerführung erscheint auf einer einzeiligen Flüssigkristallanzeige LCD-B die Aufforderung, den ersten Parameter, beispielsweise den Turgor, einzugeben. Gegebenenfalls kann dabei auch der zulässige Wertebereich angezeigt und eine Plausibilitätskontrolle des Eingabewertes durchgeführt werden. Über Nummerntasten NT wird der Kennwert eingetippt. Gleichzeitig wird der aktuelle Wert an einer neben der Flüssigkristallanzeige LCD-B angeordneten Eingabeanzeige LED-E mit Leuchtdioden angezeigt.

Durch Betätigen einer Schrittaste ST wird auf den nächsten oder den vorhergehenden Parameter (+,-) umgeschaltet. Das Löschen eines angezeigten Wertes erfolgt durch Drücken einer Korrekturtaste CT. Durch nochmaliges Drücken der Eingabetaste ET wird der Eingabemodus abgeschaltet.

Das Aktivieren der Blutsperre durch Aufpumpen der Blutsperrmanschette erfolgt mittels Betätigung einer Blutsperrtaste BST. Das Lösen der Blutsperre erfolgt durch gleichzeitiges Drücken zweier Lösetasten LT, die so weit voneinander entfernt angeordnet sind, daß dazu zwei Finger erforderlich sind. Während der Funktion der Blutsperre kann der Überdruck durch Betätigen der Schrittasten ST um einen vorgegebenen Wert erhöht oder erniedrigt (+,-) werden. Ein Tastendruck wird generell erst nach cirka 2 Sekunden registriert, um eine Fehlfunktion der Blutsperre durch irrtümliche Tastenbetätigung möglichst auszuschließen.

Störungsmeldungen, wie beispielsweise zu geringer oder zu hoher Druck in der Blutsperrmanschette, werden optisch durch ein Warnlicht WL und akustisch angezeigt. Eine Anzeige des der Störungsmeldung zugeordneten Wertes mit einem Kennungsbuchstaben erfolgt über eine Überwachungsanzeige LED-Ü, die oberhalb der Flüssigkristallanzeige LCD-B angeordnet ist. Zur besseren Auffälligkeit ist sie aus Leuchtdioden aufgebaut. Dazugehörende Kommentare oder Abhilfevorschläge werden mit der Flüssigkristallanzeige LCD-B vom Mikrocomputer ausgegeben. Wahlweise wird im Normalbetrieb an der Überwachungsanzeige LED-Ü der Druck der Blutsperrmanschette, die Dauer der Blutsperre oder die Operationszeit einzeln oder gemeinsam angezeigt. Sie kann auch für eine voreinstellbare Alarmuhr verwendet werden. Die jeweiligen Werte sind mit Buchstaben gekennzeichnet.

## Ansprüche

1. Verfahren zum Betrieb einer Blustperre für Körperextremitäten (KE), wobei der Blutdruck gemessen, dieser Meßwert digitalisiert und einem Mi-krocomputer (μC) zugeleitet wird, von dem eine Druckregelung der Blutsperre auf einen von den Parametern Gewebsturgor, Geschlecht, Extremitätenumfang und Alter der Person und Dauer der Blutsperre abhängigen Druckwert über dem aktuellen systolischen Blutdruck eingestellt wird, **dadurch gekennzeichnet,** daß die Zahlenwerte der Parameter des Druckwertes dem Mikrocompu-ter (μC) menügeführt eingegeben werden, die Blutsperre durch Betätigung einer einzigen Blutsperrtaste (BST) aktiviert und durch gleichzeitige Betätigung zweier Lösetasten (LT) beendet wird und daß Überwachungssignale und Eingabewerte mit Leuchtdioden, Bedienungshinweise alphanumerisch mit Flüssigkristallen angezeigt werden.

2. Verfahren nach Anspruch 1, **dadurch gekennzeichnet,** daß die maximale Dauer der Blutsperre ohne Schädigung des betroffenen Körpergewebes in Abhängigkeit von der Größe des Druckwertes vom Mikrocomputer (μC) berechnet und durch ein entsprechendes Überwachungssignal angezeigt wird.

3. Verfahren nach Anspruch 1 oder 2, **dadurch gekennzeichnet,** daß von einem Überwachungssignal die Aktivierung der Blutsperre angezeigt wird.

4. Verfahren nach einem der vorherigen Ansprüche, **dadurch gekennzeichnet,** daß ein Tastendruck erst nach einer vorgegebenen Mindestdauer registriert wird.

5. Vorrichtung zur Durchführung des Verfahrens nach einem der vorherigen Ansprüche, **dadurch gekennzeichnet,** daß ein Blutdruckmesser (BM) über einen Analog/Digital- Wandler (AD) mit einem Dateneingang (DE) des Mikrocomputers (μC) verbunden ist, daß der Mikrocomputer (μC) über einen Steuerausgang (SA) mit dem Ansteuerteil einer elektrisch betriebenen Pumpe (P) und über eine Datenschnittstelle (DS) mit einer Ein-/Ausgabevorrichtung (EA) verbunden ist, die aus einem Tastenfeld, einer Eingabeanzeige (LED-E) aus Leuchtdioden, einer Überwachungsanzeige (LED-Ü) aus Leuchtdioden und einer Flüssigkristallanzeige (LCD-B) für Bedienerführung und Überwachungsangaben besteht und daß der Druckteil der Pumpe (P) mit einer Blutsperrmanschette (BSM) verbunden ist.

6. Vorrichtung nach Anspruch 5, **dadurch gekennzeichnet,** daß zwei Schrittasten (ST) zur Änderung des eingestellten Überdruckes in vorgegebenen Schritten an der Ein-/Ausgabevorrichtung (EA) vorhanden sind.

7. Vorrichtung nach Anspruch 5 oder 6, **dadurch gekennzeichnet,** daß zwischen Pumpe (P) und Blutsperrmanschette (BSM) ein Rückschlagventil (RV) eingebaut ist.

8. Vorrichtung nach einem der Ansprüche 5 - 7, **dadurch gekennzeichnet,** daß zusätzlich zur Pumpe (P) eine Handpumpe (HP) mit Kontrollmanometer mit der Blutsperrmanschette (BSM) verbunden ist.

9. Vorrichtung nach einem der Ansprüche 5 - 8, **dadurch gekennzeichnet,** daß die Blutsperrmanschette (BSM) in zwei voneinander unabhängige ringförmige Kammern unterteilt ist.

10. Vorrichtung nach einem der Ansprüche 5 - 9, **dadurch gekennzeichnet,** daß ein Aufzeichnungsgerät (AG) mit einem Datenausgang (DA) des Mikrocomputers (μC) verbunden ist.

## FIG 1

| BM | | AD | DE | | SA | P | | | HP |

EA | AG

DS | DA

μC

BDM ⊢KE

KE⊣ BSM

RV

## FIG 2

LED-Ü

EA

LCD-B | LED-E

NT

☐─LT | 1 2 3

☐─BST | 4 5 6

☐─LT | 7 8 9

◯─WL | ST─ + | 0 | − ─ST

CT─ ☐ | ☐ ─ET

FIG 2